(19) Europäisches Patentamt
European Patent Office
Office européen des brevets

(11) EP 0 561 780 B1

## (12) EUROPEAN PATENT SPECIFICATION

(45) Date of publication and mention
of the grant of the patent:
**24.07.1996 Bulletin 1996/30**

(21) Application number: **91902056.0**

(22) Date of filing: **28.12.1990**

(51) Int Cl.6: **G01N 33/86**, G01N 33/92,
G01N 33/564

(86) International application number:
**PCT/US90/07688**

(87) International publication number:
**WO 92/10586 (25.06.1992 Gazette 1992/14)**

(54) **LIPID-DEPENDENT DIAGNOSTIC ASSAYS**

LIPID-ABHÄNGIGE DIAGNOSTISCHE ANALYSENVERFAHREN

ANALYSES DEPENDANT DES LIPIDES PERMETTANT D'ETABLIR UN DIAGNOSTIC

(84) Designated Contracting States:
**AT BE CH DE DK ES FR GB GR IT LI LU NL SE**

(30) Priority: **07.12.1990 US 623340**

(43) Date of publication of application:
**29.09.1993 Bulletin 1993/39**

(73) Proprietor: **THE LIPOSOME COMPANY, INC.**
**Princeton, NJ 08540 (US)**

(72) Inventors:
• JANOFF, Andrew, S.
Yardley, PA 19067 (US)
• RAUCH, Joyce
Montreal, Quebec H3X 2R8 (CA)

(74) Representative: **Warcoin, Jacques et al**
**Cabinet Régimbeau,**
**26, avenue Kléber**
**F-75116 Paris (FR)**

(56) References cited:
EP-A- 0 303 515          US-A- 4 543 339
US-A- 4 666 831          US-A- 4 698 299
US-A- 4 877 741

• TROMBOSIS AND HAEMOSTASIS vol. 62, no. 3
, 24 November 1989 pages 892 - 896 J. RAUCH
ET AL. 'DISTINGUISHING PLASMA LUPUS
ANTICOAGULANTS FROM ANTI-FACTOR
ANTIBODIES USING HEXAGONAL ( II ) PHASE
PHOSPHOLIPIDS.'
• Journal of Biological Chemistry, Volume 261,
No. 21, issued 25 July 1986, RAUCH et al.,
"Human Hybridoma Lupus Anticogulants
Distinguish between Lamellar and Hexagonal
Phase Lipid Systems", pages 9672-9677

**Description**

BACKGROUND OF THE INVENTION

1. Field of the Invention

This invention relates to diagnostic assays, and in particular to diagnostic assays for lupus anticoagulants which employ phospholipids as assay reagents.

2. Description of the Related Art

A variety of diagnostic assays are known which include one or more phospholipids as assay reagents. For example, various blood coagulation tests, such as complete and partial thromboplastin times, prothrombin times, and the like, employ brain and other tissue extracts which include lipids. Similarly, the VDRL (Venereal Disease Research Laboratory) test for syphilis is based on the use of an antigen solution which includes cardiolipin, cholesterol and lecithin.

As with many assay systems, the foregoing assays suffer from the problem of false positives, i.e., for certain patients, the coagulation tests give results indicative of a coagulation problem, when, in fact, the patient's clotting mechanisms are normal, or, in the case of the VDRL test, a patient appears to have syphilis, but is, in fact, syphilis free.

Prior studies have established a correlation between these false positives and certain diseases. For example, blood samples from patients having the autoimmune disease systemic lupus erythematosus (SLE) often have prolonged coagulation times, even though clinically the patients do not exhibit bleeding tendencies and, indeed, in some cases may suffer from thrombotic episodes. The blood of such patients is said to contain "lupus anticoagulants", "coagulation inhibitors", "lupus inhibitors", or "circulating inhibitors". See T. Exner et al., "Studies on Phospholipids in the Action of a Lupus Coagulation Inhibitor," Pathology. Vol. 7, pages 319-328 (1975); and P. Thiagarajan et al., "Monoclonal Immunoglobulin M-lambda Coagulation Inhibitor with Phospholipid Specificity," J. Clin. Invest., Vol. 66, September 1980, pages 397-405.

It is presently believed that these "inhibitors" are in fact antibodies against phospholipids which are produced by the immune system of patients suffering from SLE. See P. Thiagarajan et al., supra. Similar anti-phospholipid antibodies have been found in the sera of patients suffering from other autoimmune diseases, such as connective tissues disease, Hashimoto's thyroiditis, rheumatoid arthritis, and the like. See P. F. Sparling, "Diagnosis and Treatment of Syphilis," New England Journal of Medicine, Vol. 284, pages 642-653 (1971). Accordingly, patients with these diseases are also likely to give false positives when subjected to lipid-dependent diagnostic assays.

Efforts have been made in the past to solve the problem of false positives in lipid-dependent assays, and, in particular, lipid-dependent coagulation assays, but with only limited success. Thus, Exner et al., supra, reported that the effect of lupus inhibitor on the Russell viper venom coagulation test could be partially corrected by adding to the reagent mixture what Exner referred to as "partially characterized" phospholipids obtained from bovine cephalin using the Folch procedure. See J. Folch, "Brain Cephalin, A Mixture of Phosphatides. Separation from it of Phosphatidyl Serine, Phosphatidyl Ethanolamines, and a Fraction Containing an Inositol Phosphatide," J. Biol. Chem., Vol. 146, pages 35-41 (1942).

Exner tested three phospholipid fractions identified as phosphatidyl ethanolamine, phosphatidyl serine, and inositol phosphatide. As reported by Exner, at low concentrations each fraction reduced somewhat the clotting times of plasma samples containing lupus inhibitor, but not to the levels observed for normal samples. At higher concentrations, the addition of these phospholipid reactions unfortunately changed both the clotting times of the inhibitor-containing samples and the clotting times of the normal samples. That is, rather than solving the false positive problem, the addition of these phospholipids to the reagent mixture resulted in a change in the overall response, including the baseline, of the assay. Of the three phospholipid fractions tested, Exner stated that the phosphatidyl ethanolamine fraction appeared to give the best corrective effect.

In addition to the Exner work, Thiagarajan et al, supra, studied the effects on coagulation assays of purified IgM-lambda paraprotein obtained from a patient whose response to lipid-dependent coagulation tests indicated the presence in the patient's blood of a lupus-type anticoagulant. The purified paraprotein, when added to normal plasma, was found to reproduce the abnormal coagulation times observed with the patients plasma. Studies using the paraprotein indicated that it reacted with phosphatidylserine and phosphatidic acid, but that it did not react with phosphatidylcholine or phosphatidylethanolamine.

A comparison of the results reported by Thiagarajan with those reported by Exner highlights the confusing state of the art at that time. Whereas Thiagarajan et al. found that their lupus anticoagulant would not react with phosphatidylethanolamine, Exner et al. found just the opposite. Moreover, in Exner's hands, phosphatidylethanolamine distorted the basic character of the assay, as evidenced by the fact that the presence of 0.05% phosphatidylethanolamine in the reagent mixture resulted in an over 40% increase in the clotting time of normal plasma, and only a 30% decrease in the clotting time of a mixture of 90% normal plasma and 10% patient plasma (see Fig. 2A of Exner et al.).

Janoff et al., U.S. Patent No. 4,666,831, issued May 19, 1987, disclosed an improved lipid-dependent diagnostic assay in which the test sample to be assayed is pre-incubated with one or more phospholipids which have a hexagonal ($H_{II}$) organization when dispersed in an aqueous phase. Alternatively, Janoff et al. pre-incubate with lipidic particles. As demonstrated by the test results presented in the Janoff patent, lipid-dependent diagnostic assays which include pre-incubation with such hexagonal ($H_{II}$) phospholipids were shown to be less likely to exhibit false positives. Among the preferred phospholipids used in the Janoff patent were dioleoylphosphatidylethanolamine (DOPE) and egg phosphatidylethanolamine (EPE). A related patent, Janoff et al., U.S. Patent No. 4,698,299, issued October 6, 1987, discloses the use of bilayer-forming lysophospholipids as an alternative pre-incubation agent in such assays.

The use of such hexagonal ($H_{II}$) phospholipids in assays is further discussed in Rauch et al., "Distinguishing Plasma Lupus Anticoagulants from Anti-Factor Antibodies Using Hexagonal (II) Phase Phospholipids," <u>Thrombosis and Haemostasis</u>, 62(3) 892-896 (1989). In this article, Rauch demonstrates in a series of tests that egg phosphatidylethanolamine (EPE), inhibits the anticoagulating effects of Lupus anticoagulant without affecting the anticoagulating effects of other tested anticoagulants, such as anti-factor antibodies or heparin. Thus, hexagonal ($H_{II}$) phospholipids can be used in assays to specifically distinguish between Lupus anticoagulant and other anticoagulants.

In the assay process, as discussed in the Janoff et al. 4,666,831 patent and the Rauch et al. article, a preliminary step is the preparation of a stock solution of the $H_{II}$ phospholipid suspended in a suitable aqueous carrier such as Hepes buffer. However, the hexagonal ($H_{II}$) organization of such phospholipids which makes them suitable for use in such assays also makes them very difficult to suspend in aqueous media. This is particularly true of a pure synthetic phospholipid such as DOPE, because natural phospholipids, such as EPE, often have small amounts of impurities that may improve their suspendability. Thus when $H_{II}$ phospholipids are suspended in aqueous buffer, they have been found to precipitate quickly, thus causing problems in handling. Such mixtures need to be mixed continuously to maintain the suspensions. Furthermore, the hydrated phospholipids tend to stick to the sides of glass vessels, and are thus extremely difficult to pipette.

## SUMMARY OF THE INVENTION

In accordance with the present invention, there is provided a stable aqueous suspension of a phospholipid for use in an assay for lupus anticoagulants which is performed on a test sample, in which the assay includes the step of pre-incubating the test sample with the phospholipid, and in which the phospholipid has a hexagonal ($H_{II}$) organization when dispersed in an aqueous medium without detergent under the conditions of the assay, the suspension comprising

(a) the phospholipid;
(b) a lupus-assay compatible detergent; and
(c) an aqueous phase, wherein said phospholipid remains in suspension at a temperature of 25°C for at least one hour.

A "lupus-assay compatible detergent" is one which in combination with a selected phospholipid meets the following criteria:

1. Inhibits lupus anticoagulant specifically; and
2. Does not interfere with the anticoagulation effects of heparin, anti-Factor antibodies, and factor deficiencies.

Good results are obtained when the detergent comprises a salt of desoxycholic acid, particularly sodium desoxycholate.

In another embodiment of the present invention, there is provided an improved lipid-dependent diagnostic assay for lupus anticoagulants which is performed on a test sample, and in which the assay comprises pre-incubating the test sample with one or more phospholipids which have a hexagonal ($H_{II}$) organization when dispersed in an aqueous phase without detergent under the conditions of the assay, the improvement comprising:

providing said phospholipids in combination with a lupus-assay compatible detergent, the combination of phospholipid and detergent forming a stable aqueous suspension when mixed with said aqueous phase under the conditions of the assay, in which suspension said phospholipids can remain in suspension at a temperature of 25°C for at least one hour; and

pre-incubating the test sample with said combination of phospholipid and lupus-assay compatible detergent.

In particular embodiments, the phospholipids are dioleoylphosphatidylethanolamine, egg phosphatidylethanolamine or bovine phosphatidylethanolamine. In other particular embodiments, the detergent comprises a salt of desoxycholic acid, with good results obtained using sodium desoxycholate.

In a further aspect of the present invention, there is provided an improved assay for use in determining whether a patient has lupus anticoagulants wherein the assay comprises the steps of:

(a) obtaining first and second samples of the patient's plasma;
(b) incubating the first sample with one or more phospholipids which have a hexagonal ($H_{II}$) organization when dispersed in an aqueous medium without detergent under the conditions of the assay;
(c) performing a lipid-dependent diagnostic assay on both the first and second samples, the assay producing a positive reading when used to assay a sample which contains lupus anticoagulants; and
(d) comparing the results of the assays performed on the first and second samples, the presence of a normal result for the first sample and a positive result for the second sample being indicative of the patient having lupus anticoagulants; the improvement comprising:

providing the phospholipids in combination with a lupus-assay compatible detergent which can form a stable aqueous suspension comprising an aqueous phase, the phospholipids and a detergent, in which suspension said phospholipids can remain in suspension at a temperature of 25°C for at least one hour.

Applicants have found that phospholipids which normally have a hexagonal ($H_{II}$) organization when dispersed in an aqueous medium without detergent can be made into stable aqueous suspensions by combining them with a detergent, such as a desoxycholate, and that this can be done without affecting the ability of such phospholipids to be used in lipid-dependent diagnostic assays for lupus anticoagulants, as discussed above. This result is particularly surprising, because the detergents appear to alter, at least partially, the normal hexagonal ($H_{II}$) organization of the phospholipids without significantly affecting the actions of such phospholipids in lipid-dependent assays. The ability to formulate such phospholipids into stable aqueous suspensions for use in assay procedures greatly facilitates the use of these phospholipids.

DETAILED DESCRIPTION

As described above, in accordance with a particular embodiment of the present invention, there is provided a stable aqueous suspension of a phospholipid for use in an assay for lupus anticoagulants which is performed on a test sample, in which the assay includes the step of pre-incubating the test sample with the phospholipid, and in which the phospholipid has a hexagonal ($H_{II}$) organization when dispersed in the test sample without detergent under the conditions of the assay, the suspension comprising

(a) the phospholipid;
(b) a lupus-assay compatible detergent; and
(c) an aqueous phase,

wherein said phospholipid remains in suspension at a temperature of 25°C for at least one hour.

The phospholipid is one which has a hexagonal ($H_{II}$) organization when dispersed in an aqueous medium without detergent under the conditions of the assay. Such "conditions of the assay" include, but are not limited to, temperature, concentration of all components, ionic concentrations, pH, etc. Many such assays are conducted at 37°C, and therefore for such assays the phospholipid must be in $H_{II}$ form at that temperature. Examples of phospholipids which are suitable for use in the present invention include dioleoylphosphatidylethanolamine (DOPE), egg phosphatidylethanolamine (EPE), and bovine phosphatidylethanolamine and certain phophatidic acids. In addition, cardiolipin can be in hexagonal ($H_{II}$) phase when provided in combination with calcium ions, as is well known in the art. Of these, DOPE is particularly suitable because it is a synthetic phospholipid and thus generally freer of contaminants than the phospholipids derived from natural sources. As discussed above, it is the purity of DOPE that has also made it particularly hard to maintain in suspension and handle in assay procedures.

For purposes of this application, the stable aqueous suspensions are defined as "stable" when they stay in suspension at room temperature (25°C) for a period of at least one hour. In fact, the suspensions used in the Examples below were found to be stable for periods of four hours and even longer. Conversely, the mixtures of hexagonal ($H_{II}$) phospholipids in buffer solution without detergent were found to settle out of suspension almost immediately after cessation of agitation. Therefore, the one-hour limit clearly differentiates the suspensions of the present invention from $H_{II}$ phospholipid mixtures without detergent.

The detergents suitable for use in the present invention must be lupus-assay compatible, as previouly defined, and must be able to form a stable aqueous suspension with a phospholipid. The suitability of a particular detergent for use with a selected phospholipid in the performance of a particular assay should be readily determinable by one skilled in the art. A preferred detergent for use in the present invention is a salt of desoxycholic acid, particularly sodium desoxycholate, which is used in the Examples below. Good results were obtained using about 40 to 50 mol % detergent, based on the total amount of combined phospholipid and detergent.

The $H_{II}$ phospholipid suspension is used to pre-incubate a test sample for a lipid-dependent diagnostic assay for lupus anticoagulants. This process is described in detail in the Janoff et al. 4,666,831 patent, cited above. A particular assay to which the present invention is applicable are coagulation tests on human plasma as part of an activated partial thromboplastin time (APTT) assay. The present invention may also be applicable to other lipid dependent diagnostic assays such as prothrombin times, Russell viper venom times, Taipan snake venom times, and cardiolipin-dependent tests for syphilis, e.g., the VDRL test.

The stable aqueous phospholipid suspension of the present invention is of particular use in assays for determining whether a patient has lupus anticoagulants. Such assays comprise the steps of:

(a) obtaining first and second samples of the patient's plasma;
(b) incubating the first sample with a stable aqueous phospholipid suspension made in accordance with the present invention;
(c) performing a lipid-dependent diagnostic assay on both the first and second samples, the assay producing a positive reading when used to assay a sample which contains anti-phospholipid antibodies; and
(d) comparing the results of the assays performed on the first and second samples, the presence of a normal result for the first sample and a positive result for the second sample being indicative of the patient having lupus anticoagulants.

In a particular embodiment of the present invention, the assay for lupus anticoagulants is a human plasma coagulation test which measures activated partial thromboplastin time (APTT). In the Examples which follow, a stabilized aqueous suspension of DOPE in combination with sodium desoxycholate is used to pre-incubate samples for APTT assay. The materials and methods common to the various examples were as follows.

MATERIALS AND METHODS

Preparation of Test Materials

Dioleoylphosphatidylethanolamine (DOPE) was purchased from Avanti Polar Lipids (Birmingham, Alabama). A stock solution was prepared of 50 mg/ml DOPE in $CHCl_3$ (chloroform).

Sodium desoxycholate was obtained from Fisher Scientific Co. (Montreal, Quebec). The desoxycholate was made into a stock solution of 50 mg/ml in 1:1 $CHCl_3$:MeOH (chloroform:methanol).

A stock suspension of DOPE (4 mg/ml)-sodium desoxycholate (50 mol %) was made as follows: 40 µl of the DOPE stock solution and 22 µl of the desoxycholate stock solution were placed in a round bottom glass tube and an additional 40 µl of 1:1 $CHCl_3$:MeOH was added. The solvents were then removed by evaporation under dry nitrogen for 30 minutes. The dried residue was then resuspended in 0.5 ml of 20 mM Hepes buffer (15 mM NaCl, pH 7.5), and hydrated for 15 minutes at a temperature of 37°C, and then for 45 minutes at 25°C. These temperatures are well above the lamellar to hexagonal ($H_{II}$) transition temperature of DOPE, which is less than about 10°C. The Hepes buffer was obtained from Sigma Chemical Co., St. Louis, Missouri.

A stock solution of sodium desoxycholate in Hepes buffer was also prepared for use in the comparative tests. The desoxycholate/Hepes stock solution was prepared by mixing 22 ul of the desoxycholate stock solution with an additional 22 ul 1:1 $CHCl_3$:MeOH, and then removing the solvents by evaporation under dry nitrogen. Then 0.5 ml of 20 mM Hepes buffer (15 mM NaCl, pH 7.5) was added, and the composition mixed to form the desoxycholate/Hepes stock solution.

Activated partial thromboplastin time (APTT) reagent, a platelet factor 3 reagent (partial thromboplastin) plus particulate activator, was obtained from Organon Teknika, Inc., Scarborough, Ontario (hereinafter referred to as "Organon"). The reagent was diluted 1/32 in 20 mM Hepes buffer (15 mM NaCl, pH 7.5).

Human Plasmas

Blood was drawn into blue stopper blood collection tubes (4.5 ml total volume/tube) (Vacutainer Systems, Rutherford, NJ) containing 0.5 ml 3.8% buffered sodium citrate. The blood was then centrifuged for 30 minutes at 1800 rpm (800xg) at room temperature on an IEC tabletop centrifuge to obtain platelet-poor plasma. Verify normal citrated plasma (pooled normal human plasma) was obtained from Organon. Lupus anticoagulant-containing plasmas were obtained from 10 patients with systemic lupus erythematosus (SLE) from the McGill Lupus Registry. All patients fulfilled the 1982 revised American Rheumatism Association criteria for the classification of SLE. A circulating lupus anticoagulant was considered to be present if the APTT was ≥5 sec. above the normal plasma control and a 1:1 mixture of patient's plasma with normal plasma did not correct the prolonged APTT in a dilute APTT assay. Two lupus anticoagulant-containing plasmas were also obtained from SLE patients receiving intravenous heparin therapy.

Factor deficient plasmas were obtained in lyophilized form from Organon and in fresh frozen form from the Coagulation Laboratory of Montreal General Hospital. Anti-factor antibody-containing plasmas from hemophiliac patients with anti-Factor VIII antibodies were supplied by Montreal Children's Hospital. Plasmas of patients receiving intravenous heparin therapy were provided by the Coagulation Laboratory of Montreal General Hospital. All plasmas were frozen at -70°C until required for assay.

Phospholipid Inhibition of the Diluted APTT Assay

Clotting times were determined using the General Diagnostics Coag-a-mate (R) ISO single channel instrument (Warner-Lambert Co., Morris Plains, NJ), a semi-automated photo-optical clot detection system. Seventy-five µl of plasma was mixed with an equal volume of of the stock suspension of DOPE (4mg/ml) - 50 mol % desoxycholate in Hepes buffer and incubated for 10 min. in a 37°C water bath. Fifty ul of this mixture was then diluted with an equal volume of freshly reconstituted Verify normal citrated plasma in a cuvette in a disposable circular test tray (obtained from Organon). One hundred ul of the 1/32 dilution of APTT reagent, which had been prewarmed to 37°C, was then added and the mixture incubated for 5 min. at 37°C.

After incubation of the sample, 100 ul of 25 mM $CaCl_2$ was added to the cuvette through the reagent incubation arm of the Coag-a-mate (R) machine, initiating the clotting sequence. The clotting time was displayed on the Coag-a-mate (R) digital timer and recorded. All samples were tested in duplicate and on two separate occasions to ensure reproducibility. The ability of the stock DOPE-desoxycholate suspension ("DOPE-desoxy") to inhibit anticoagulant activity when compared to stock desoxycholate/Hepes solution ("desoxy") was calculated using the following formula, designated Equation 1. (In those cases in which comparative stock desoxycholate/Hepes solutions were not tested, as indicated by "nt" in the test results, the plasma + buffer APTT value was used in place of the plasma + desoxy APTT value):

$$ \% \text{ Inhibition} = \frac{\text{APTT (plasma + desoxy) - APTT (plasma + DOPE-desoxy)}}{\text{APTT (plasma + desoxy) - APTT (Verify + DOPE-desoxy)}} \times 100\% $$

EXAMPLE 1

EFFECT OF DOPE-DESOXYCHOLATE SUSPENSION ON LUPUS ANTICOAGULANT ACTIVITY IN SLE PLASMA

Five samples of plasma containing lupus anticoagulant (LA) and one Verify sample were tested by the APTT assay. Each sample was tested after incubation with 1) a Hepes buffer control ("Hepes"); 2) the stock desoxycholate/Hepes solution ("desoxy") (samples 1 to 3 only); and 3) the stock DOPE-desoxycholate suspension ("DOPE-desoxy"), respectively. For each sample, the clotting time after incubation in the DOPE-desoxy suspension was compared to the clotting time after incubation in desoxy/Hepes (samples 1-3) or plain Hepes buffer (samples 4-5), to determine the % inhibition of the APTT test, calculated in accordance with Equation 1 above. The results are presented in Table I.

TABLE I

| Plasma Sample | APTT (sec) | | | % Inhibition of APTT |
|---|---|---|---|---|
| | +Hepes | +Desoxy | +Dope-Desoxy | |
| Verify LA | 39.0 | 38.8 | 40.2 | |
| 1 | 57.4 | 56.3 | 42.3 | 87.0 |
| 2 | 59.7 | 58.9 | 43.5 | 82.3 |
| 3 | 66.5 | 64.1 | 45.2 | 94.5 |
| 4 | 53.4 | nt | 44.4 | 89.1 |
| 5 | 64.6 | nt | 44.4 | 94.8 |

nt - not tested

As would be expected, the lupus anticoagulant in Hepes buffer greatly prolonged the clotting time of the plasma sample in the APTT test. Incubation with the desoxycholate/Hepes solution had only a negligible effect on clotting times when compared to the Hepes buffer controls. On the other hand, incubation with the DOPE-desoxycholate suspension significantly inhibited 80 to 95 percent of the effect of the lupus anticoagulant, so that the coagulation times were only slightly longer than that of the Verify sample.

EXAMPLE 2

EFFECT OF DOPE-DESOXYCHOLATE SUSPENSION ON ANTI-FACTOR ANTIBODY PLASMAS

Five samples of plasma containing anti-Factor VIII and one Verify sample were tested by APTT assay following the same procedure used in Example 1. The results are presented in Table II:

TABLE II

| Plasma Sample | APTT (sec) | | | % Inhibition of APTT |
|---|---|---|---|---|
| | +Hepes | +Desoxy | +Dope-Desoxy | |
| Verify A-F VIII | 39.8 | 40.4 | 42.5 | |
| 1 | 51.5 | 50.1 | 47.9 | 22.6 |
| 2 | 65.4 | 61.3 | 67.1 | 0 |
| 3 | 66.8 | 64.9 | 59.8 | 24.5 |
| 4 | 49.0 | nt | 49.5 | 0 |
| 5 | 49.6 | nt | 50.2 | 0 |

nt - not tested

As in Example 1, the results in Table II show that anti-Factor VIII samples had significantly prolonged APTT coagulation times, as compared to the Verify sample. However, in this case, incubation with the DOPE-desoxycholate suspension had little or no effect on the prolonging of coagulation time caused by anti-Factor VIII. This demonstrates the selectivity of the DOPE-desoxycholate for lupus anticoagulant as opposed to other anticoagulant factors. Again, incubation with desoxycholate/Hepes solution was found to have only a negligible inhibitory effect on the prolonged coagulation times.

EXAMPLE 3

EFFECT OF DOPE-DESOXYCHOLATE SUSPENSION ON FACTOR DEFICIENT PLASMAS

Six samples of plasmas deficient in particular clotting factors and one Verify sample were tested by the procedure of Example 1. Each sample was composed of 80% of the specified plasma and 20% of the Verify plasma. Tests were conducted on factor-deficient plasmas obtained from patients (labeled "F." factor numbers in the following table) and on commercial factor deficient plasmas obtained from Organon (labeled "C. F." factor numbers in the table). The results are presented in Table III:

TABLE III

| Plasma Sample | APTT (sec) | | | % Inhibition of APTT |
|---|---|---|---|---|
| | +Hepes | +Desoxy | +Dope-Desoxy | |
| Verify | 39.4 | 39.0 | 40.0 | |
| F. XII | 48.1 | 49.8 | 47.1 | 27.5 |
| C. F. VIII | 67.9 | 68.6 | 61.4 | 25.2 |
| F. XI | 79.3 | nt | 72.8 | 16.5 |
| C. F. XI | 70.8 | 64.9 | 65.5 | 0 |
| C. F. XII | 59.8 | 55.3 | 55.9 | 0 |
| C. F. IX | 69.2 | 69.8 | 68.4 | 4.7 |

nt - not tested

As in the previous examples, the plasma deficient samples all showed prolonged APTT clotting times as compared to the Verify sample. For these factor deficient samples, incubation with the DOPE-desoxycholate suspension also showed negligible effect on the clotting time. In each case, incubation with the desoxycholate/Hepes solution showed only negligible effect on the APTT time. Again, this shows how DOPE in combination with desoxycholate can be used to distinguish clotting inhibition caused by lupus anticoagulant from that caused by a deficiency of a particular clotting factor, and that the desoxycholate does not interfere with this determination.

7

EXAMPLE 4

EFFECT OF DOPE-DESOXYCHOLATE SUSPENSION ON HEPARINIZED PLASMAS

In this example, four samples of heparinized plasmas, a fifth heparinized lupus anticoagulant-containing plasma, and a Verify sample were tested in accordance with the procedure of Example 1. The results are presented in Table IV:

TABLE IV

| Plasma Sample | APTT (sec) | | | % Inhibition of APTT |
|---|---|---|---|---|
| | +Hepes | +Desoxy | +Dope-Desoxy | |
| Verify | 37.5 | 37.6 | 40.8 | |
| Hep. 1 | 45.1 | 54.2 | 54.3 | 0 |
| Hep. 2 | 47.1 | 55.4 | 56.6 | 0 |
| Hep. 3 | 50.6 | 50.5 | 51.4 | 0 |
| Hep. 4 | 51.6 | 51.7 | 53.0 | 0 |
| LA + Hep. | 60.1 | 60.4 | 54.5 | 20.9 |

The test results show the normal anticoagulation effect of heparin on plasma samples. In this example, the pre-incubation of heparinized samples with desoxycholate/Hepes solution or with DOPE-desoxycholate suspension was found to have no inhibitory effect on the clotting prolongation caused by the heparin. In fact, the pre-incubations even caused a further prolonging of clotting times in some samples. In the last sample, clotting was prolonged by both heparin and lupus anticoagulant. In this case, the DOPE-desoxycholate pre-incubated sample showed some inhibition of the clotting time prolongation, while the desoxycholate pre-incubated sample showed no such inhibition. This demonstrates the selectivity of the DOPE-desoxycholate for lupus anticoagulant, as opposed to heparin anticoagulant, and also demonstrates that desoxycholate by itself does not affect the lupus anticoagulant clotting prolongation.

EXAMPLE 5

DRIED-DOWN DOPE-DESOXYCHOLATE

In this example, 38 $\mu$l of the 50 mg/ml desoxycholate stock solution were combined with 90 $\mu$l of the 50 mg/ml DOPE stock solution. Additional 1:1 $CHCl_3$:MeOH solvent was added to make up 1 ml of solution. Then 0.1 ml of this solution was pipetted into ten individual 12 x 75 mm test-tubes, and the solvent was removed by evaporation under dry nitrogen for 30 minutes. This left a dried-down coating of DOPE in combination with the desoxycholate on the inside of the test-tubes (hereinafter referred to as "DOPE-desoxycholate tubes"). (These Dope-desoxycholate tubes can be used immediately or frozen and stored for future use at, for example, -70°C.) For comparative tests with desoxycholate, 38 ul of the 50 mg/ml desoxycholate stock solution was mixed with sufficient 1:1 $CHCl_3$:MeOH solvent to make up 1 ml of solution. Then 0.1 ml aliquots of this solution were pipetted into ten test-tubes and the tubes dried down as above (hereinafter referred to as "desoxycholate tubes"). In this manner, a supply of tubes containing dried-down DOPE-desoxycholate and dried-down desoxycholate were prepared for the following APTT assay tests.

In the following tests, 150 $\mu$l samples of each plasma were added to an empty tube (identified as "neat" in the test results), a desoxycholate tube, and a DOPE-desoxycholate tube, respectively. In these samples, it is the plasma itself which is the aqueous medium in which the DOPE-desoxycholate combination is suspended. These DOPE-desoxycholate samples contained 3 mg/ml DOPE and 43 mol % desoxycholate. All the samples were pre-incubated for 15 minutes in a 37°C water bath.

APTT assays were then conducted on the samples by the process described above. Fifty $\mu$l of each sample was diluted with an equal volume of freshly reconstituted Verify normal citrated plasma in a cuvette in a disposable circular test tray (obtained from Organon). One hundred ul of the 1/32 dilution of APTT reagent, which had been prewarmed to 37°C, was then added and the mixture incubated for 5 min. at 37°C. The results of the tests are presented in Table V:

TABLE V

| Plasma Sample | APTT (sec) | | | % Inhibition of APTT |
|---|---|---|---|---|
| | +Hepes | +Desoxy | +Dope-Desoxy | |
| Verify | 39.3 | 36.2 | 40.4 | |

TABLE V (continued)

| Plasma Sample | APTT (sec) | | | % Inhibition of APTT |
|---|---|---|---|---|
| | +Hepes | +Desoxy | +Dope-Desoxy | |
| LA 1 | 61.5 | 57.5 | 43.3 | 83.0 |
| 2 | 57.5 | 57.5 | 40.7 | 98.2 |
| 3 | 52.7 | 49.8 | 39.6 | 100 |
| Anti-Factor VIII | | | | |
| 1 | 50.1 | 50.1 | 51.9 | 0 |
| 2 | 57.0 | 56.1 | 56.2 | 0 |
| 3 | 45.0 | 44.3 | 45.6 | 0 |
| 4 | 45.9 | 45.0 | 46.2 | 0 |
| Factor Deficient | | | | |
| C. F. XI | 67.8 | 66.0 | 64.7 | 5.0 |
| C. F. XII | 59.4 | 57.2 | 57.3 | 0 |
| Hep. 1 | 62.0 | 60.5 | 62.6 | 0 |
| 2 | 51.4 | 50.5 | 51.4 | 0 |
| 3 | 57.0 | 56.5 | 62.4 | Prolonged |
| 4 | 50.2 | 53.6 | 71.0 | Prolonged |

As with Examples 1 to 4, the results in the above Table V show the selectivity of the DOPE-desoxycholate pre-incubation for inhibiting the prolongation of the clotting in the APTT assay caused by lupus anticoagulant as opposed to other anticoagulant factors.

The terms and expressions which have been employed are used as terms of description and not of limitation, and there is no intention in the use of such terms and expression of excluding any equivalents of the features described or portions thereof, but it is recognized that various modifications are possible within the scope of the invention claimed.

**Claims**

**Claims for the following Contracting States : AT, BE, CH, LI, DE, DK, FR, GB, GR, IT, LU, NL, SE**

1. A stable aqueous suspension of a phospholipid for use in an assay for lupus anticoagulants which is performed on a test sample, in which the assay includes the step of pre-incubating the test sample with the phospholipid, and in which the phospholipid has a hexagonal ($H_{II}$) organization when dispersed in an aqueous medium without detergent under the conditions of the assay, the suspension comprising

    (a) the phospholipid;
    (b) a lupus-assay compatible detergent; and
    (c) an aqueous phase,

    wherein said phospholipid remains in suspension at a temperature of 25°C for at least one hour.

2. The stable suspension of claim 1 wherein the phospholipid is selected from the group consisting of dioleoylphosphatidylethanolamine, egg phosphatidylethanolamine, and bovine phosphatidylethanolamine.

3. The stable suspension of claim 1 wherein said lupus assay compatible detergent comprises a salt of desoxycholic acid.

4. The stable suspension of claim 3 wherein said salt is sodium desoxycholate.

**5.** The stable suspension of claim 4 wherein the phospholipid is dioleoylphosphatidylethanolamine.

**6.** The stable suspension of claim 1 wherein said aqueous phase is an aqueous buffer solution.

**7.** A lipid-dependent diagnostic assay for lupus anticoagulants which is performed on a test sample, and in which the assay comprises pre-incubating the test sample with one or more phospholipids which have a hexagonal ($H_{II}$) organization when dispersed in an aqueous phase without detergent under the conditions of the assay, comprising:

providing said phospholipids in combination with a lupus-assay compatible detergent, the combination of phospholipid and detergent forming a stable aqueous suspension when mixed with said aqueous phase under the conditions of the assay, in which suspension said phospholipids can remain in suspension at a temperature of 25°C for at least one hour; and
pre-incubating the test sample with said combination of phospholipid and lupus-assay compatible detergent.

**8.** An assay for use in determining whether a patient has lupus anticoagulants wherein the assay comprises the steps of:

(a) obtaining first and second samples of the patient's plasma;
(b) incubating the first sample with one or more phospholipids which have a hexagonal ($H_{II}$) organization when dispersed in an aqueous medium without detergent under the conditions of the assay;
(c) performing a lipid-dependent diagnostic assay on both the first and second samples, the assay producing a positive reading when used to assay a sample which contains lupus anticoagulants; and
(d) comparing the results of the assays performed on the first and second samples, the presence of a normal result for the first sample and a positive result for the second sample being indicative of the patient having lupus anticoagulants; the improvement comprising:
providing the phospholipids in combination with a lupus-assay compatible detergent which can form a stable aqueous suspension comprising an aqueous phase, the phospholipids and a detergent, in which suspension said phospholipids can remain in suspension at a temperature of 25°C for at least one hour.

**9.** The assay of claim 7 or 8 wherein the test sample is human plasma and the assay is a coagulation test.

**10.** The assay of claim 9 wherein the assay is an assay measuring activated partial thromboplastin time.

**11.** The assay of claim 7 or 8 wherein the phospholipids are selected from the group consisting of dioleoylphospbatidylethanolamine, egg phosphatidylethanolamine, and bovine phosphatidylethanolamine.

**12.** The assay of claim 11 wherein the detergent comprises a salt of desoxycholic acid.

**13.** The assay of claim 12 wherein the salt is sodium desoxycholate.

**14.** The assay of claim 13 wherein the phospholipid is dioleoylphosphatidylethanolamine.


**Claims for the following Contracting State : ES**

**1.** A method for preparing a stable aqueous suspension of a phospholipid for use in an assay for lupus anticoagulants which is performed on a test sample, in which the assay includes the step of pre-incubating the test sample with the phospholipid, and in which the phospholipid has a hexagonal ($H_{II}$) organization when dispersed in an aqueous medium without detergent under the conditions of the assay, the method comprising the admixture of:

a) the phospholipid ;
b) a lupus-assay compatible detergent ; and
c) an aqueous phase,

wherein said phospholipid remains in suspension at a temperature of 25°C for at least one hour.

**2.** The method of claim 1, wherein the phospholipid is selected from the group consisting of dioleoylphosphatidylethanolamine, egg phosphatidylethanolamine, and bovine phosphatidylethanolamine.

3. The method of claim 1, wherein said lupus assay compatible detergent comprises a salt of desoxycholic acid.

4. The method of claim 3, wherein said salt is sodium desoxycholate.

5. The method of claim 4, wherein the phospholipid is dioleoylphosphatidylethanolamine.

6. The method of claim 1, wherein said aqueous phase is an aqueous buffer solution.

7. A lipid-dependent diagnostic assay for lupus anticoagulants which is performed on a test sample, and in which the assay comprises pre-incubating the test sample with one or more phospholipids which have a hexagonal ($H_{II}$) organization when dispersed in an aqueous phase without detergent under the conditions of the assay comprising :

- providing said phospholipids in combination with a lupus-assay compatible detergent, the combination of phospholipid and detergent forming a satble aqueous suspension when mixed with said aqueous phase under the conditions of the assay, in which suspension said phospholipids can remain in suspension at a temperature of 25°C for at least one hour ; and
- pre-incubating the test sample with said combination of phospholipid and lupus-assay compatible detergent.

8. An assay for use in determining whether a patient has lupus anticoagulants, wherein the assay comprises the steps of :

a) obtaining first and second samples of the patient's plasma ;
b) incubating the first sample with one or more phospholipids which have a hexagonal ($H_{II}$) organization when dispersed in an aqueous medium without detergent under the conditions of the assay ;
c) performing a lipid-dependent diagnostic assay on both the first and second samples, the assay producing a positive reading when used to assay a sample which contains lupus anticoagulants ; and
d) comparing the results of the assays performed on the first and second samples, the presence of a normal result for the first sample and a positive result for the second sample being indicative of the patient having lupus anticoagulants ; the improvement comprising :

- providing the phospholipids in combination with a lupus-assay compatible detergent which can form a stable aqueous suspension comprising an aqueous phase, the phospholipids and a detergent, in which suspension said phospholipids can remain in suspension at a temperature of 25°C for at least one hour.

9. The assay of claim 7 or 8, wherein the test sample is human plasma and the assay is a coagulation test.

10. The assay of claim 9, wherein the assay is an assay measuring activated partial thromboplastin time.

11. The assay of claim 7 or 8, wherein the phospholipids are selected from the group consisting of dioleoylphosphatidylethanolamine, egg phosphatidylethanolamine, and bovine phosphatidylethanolamine.

12. The assay of claim 11, wherein the detergent comprises a salt of desoxycholic acid.

13. The assay of claim 12, wherein the salt is sodium desoxycholate.

14. The assay of claim 13, wherein the phospholipid is dioleoylphosphatidylethanolamine.


**Patentansprüche**


**Patentansprüche für folgende Vertragsstaaten : AT, BE, CH, LI, DE, DK, FR, GB, GR, IT, LU, NL, SE**

1. Stabile wäßrige Suspension eines Phospholipids zur Verwendung in einem an einer Testprobe durchgeführten Verfahren zum Nachweis von Lupusantikoagulanzien, dadurch gekennzeichnet, daß das Nachweisverfahren den Schritt einer Vorinkubation der Testprobe mit dem Phospholipid umfaßt und daß das Phospholipid eine hexagonale ($H_{II}$) Anordnung aufweist, wenn es unter den Testbedingungen in einem wäßrigen Medium ohne Detergens suspendiert wird, und wobei die Suspension

EP 0 561 780 B1

(a) das Phospholipid,
(b) ein für Lupustests geeignetes Detergens und
(c) eine wäßrige Phase

umfaßt, in der das Phospholipid bei einer Temperatur von 25°C mindestens eine Stunde lang suspendiert bleibt.

2. Stabile Suspension nach Anspruch 1, dadurch gekennzeichnet, daß das Phospholipid aus der Dioleoylphosphatidylethanolamin, Eier-Phosphatidylethanolamin und Rinder-Phosphatidylethanolamin umfassenden Gruppe ausgewählt wird.

3. Stabile Suspension nach Anspruch 1, dadurch gekennzeichnet, daß das für Lupustests geeignete Detergens ein Salz der Desoxycholsäure enthält.

4. Stabile Suspension nach Anspruch 3, dadurch gekennzeichnet, daß das Salz Natriumdesoxycholat ist.

5. Stabile Suspension nach Anspruch 4, dadurch gekennzeichnet, daß das Phospholipid Dioleoylphosphatidylethanolamin ist.

6. Stabile Suspension nach Anspruch 1, dadurch gekennzeichnet, daß die wäßrige Phase eine wäßrige Pufferlösung ist.

7. Lipidabhängiger diagnostischer, an einer Testprobe durchgeführter Test auf Lupusantikoagulanzien, dadurch gekennzeichnet, daß der Test die Vorinkubation der Testprobe mit einem oder mehreren Phospholipiden umfaßt, die eine hexagonale ($H_{II}$) Anordnung aufweisen, wenn sie unter den Testbedingungen in einer wäßrigen Phase ohne Detergens dispergiert werden, und folgendes umfassend:

Bereitstellung der besagten Phospholipide in Kombination mit einem für Lupustests geeigneten Detergens, wobei die Verbindung aus Phospholipid und Detergens eine stabile wäßrige Suspension bildet, wenn sie unter den Testbedingungen mit besagter wäßriger Phase gemischt wird, und in welcher Suspension die besagten Phospholipide bei einer Temperatur von 25°C mindestens eine Stunde lang suspendiert bleiben können; und Vorinkubation der Testprobe mit besagter Kombination aus Phospholipid und für Lupustests geeignetem Detergens.

8. Verfahren zum Nachweis von Lupusantikoagulanzien bei einem Patienten, dadurch gekennzeichnet, daß der Test die folgenden Schritte umfaßt:

(a) Einholung einer ersten und zweiten Plasmaprobe vom Patienten;
(b) Inkubation der ersten Probe mit einem oder mehreren Phospholipiden, die eine hexagonale ($H_{II}$) Anordnung aufweisen, wenn sie unter den Testbedingungen in einem wäßrigen Medium ohne Detergens dispergiert werden;
(c) Durchführung eines lipidabhängigen diagnostischen Tests an sowohl der ersten als auch der zweiten Probe, wobei der Test ein positives Ergebnis liefert, wenn eine Lupusantikoagulanzien enthaltende Probe getestet wird; und
(d) Vergleich der Testergebnisse für die erste und die zweite Probe, wobei das Vorliegen eines normalen Ergebnisses für die erste Probe und eines positiven Ergebnisses für die zweite Probe ein Anzeichen für die Anwesenheit von Lupusantikoagulanzien beim Patienten darstellt; wobei die Verbesserung aus folgendem besteht:

Bereitstellung der Phospholipide in Kombination mit einem für Lupustests geeigneten Detergens, die eine stabile wäßrige Suspension mit einer wäßrigen Phase, den Phospholipiden und einem Detergens bilden kann, und in welcher Suspension die besagten Phospholipide bei einer Temperatur von 25°C mindestens eine Stunde lang suspendiert bleiben können.

9. Nachweisverfahren nach Anspruch 7 oder 8, dadurch gekennzeichnet, daß die Testprobe menschliches Plasma und das Nachweisverfahren ein Gerinnungstest ist.

10. Nachweisverfahren nach Anspruch 9, dadurch gekennzeichnet, daß das Nachweisverfahren ein Verfahren zur Messung der aktivierten partiellen Thromboplastinzeit ist.

**11.** Nachweisverfahren nach Anspruch 7 oder 8, dadurch gekennzeichnet, daß die Phospholipide aus der Dioleoylphosphatidylethanolamin, Eier-Phosphatidylethanolamin und Rinder-Phosphatidylethanolamin umfassenden Gruppe ausgewählt werden.

**12.** Nachweisverfahren nach Anspruch 11, dadurch gekennzeichnet, daß das Detergens ein Salz der Desoxycholsäure enthält.

**13.** Nachweisverfahren nach Anspruch 12, dadurch gekennzeichnet, daß das Salz Natriumdesoxycholat ist.

**14.** Nachweisverfahren nach Anspruch 13, dadurch gekennzeichnet, daß das Phospholipid Dioleoylphosphatidylethanolamin ist.


**Patentansprüche für folgenden Vertragsstaat : ES**

**1.** Verfahren zum Nachweis von Lupusantikoagulanzion in einer Testprobe mit hilfe einer stabilen wässrigen Suspension dadurch gekennzeichnet, daß das Nachweisverfahren den Schritt einer Vorinkubation der Testprobe mit dem Phospholipid umfaßt und daß das Phospholipid eine hexagonale ($H_{II}$) Anordnung aufweist, wenn es unter den Testbedingungen in einem wäßrigen Medium ohne Detergens suspendiert wird, und wobei die Suspensionen

    (a) das Phospholipid,
    (b) ein für Lupustests geeignetes Detergens und
    (c) eine wäßrige Phase

umfaßt, in der das Phospholipid bei einer Temperatur von 25°C mindestens eine Stunde lang suspendiert bleibt.

**2.** Verfahren nach Anspruch 1, dadurch gekennzeichnet, daß das Phospholipid aus der Dioleoylphosphatidylethanolamin, Eier-Phosphatidylethanolamin und Rinder-Phosphatidylethanolamin umfassenden Gruppe ausgewählt wird.

**3.** Verfahren nach Anspruch 1, dadurch gekennzeichnet, daß das für Lupustests geeignete Detergenz ein Salz der Desoxycholsäure enthält.

**4.** Verfahren nach Anspruch 3, dadurch gekennzeichnet, daß das Salz Natriumdesoxycholat ist.

**5.** Verfahren nach Anspruch 4, dadurch gekennzeichnet, daß das Phospholipid Dioleoylphosphatidylethanolamin ist.

**6.** Verfahren nach Anspruch 1, dadurch gekennzeichnet, daß die wäßrige Phase eine wäßrige Pufferlösung ist.

**7.** Lipidabhängiger diagnostischer, an einer Testprobe durchgeführter Test auf Lupusantikoagulanzien, dadurch gekennzeichnet, daß der Test die Vorinkubation der Testprobe mit einem oder mehreren Phospholipiden umfaßt, die eine hexagonale ($H_{II}$) Anordnung aufweisen, wenn sie unter den Testbedingungen in einer wäßrigen Phase ohne Detergens dispergiert werden, und folgendes umfassen:

    Bereitstellung der besagten Phospholipide in Kombination mit einem für Lupustests geeigneten Detergens, wobei die Kombination aus Phospholipid und Detergens eine stabile wäßrige Suspension bildet, wenn sie unter den Testbedingungen mit besagter wäßriger Phase gemischt wird, und wobei die besagten Phospholipide in der Suspension bei einer Temperatur von 25°C mindestens eine Stunde lang suspendiert bleiben können; und

    Vorinkubation der Testprobe mit besagter Kombination aus Phospholipid und für Lupustests geeignetem Detergens.

**8.** Verfahren zum Nachweis von Lupusantikoagulanzien bei einem Patienten, dadurch gekennzeichnet, daß der Test die folgenden Schritte umfaßt:

    (a) Einholung einer ersten und zweiten Plasmaprobe vom Patienten;
    (b) Inkubation der ersten Probe mit einem oder mehreren Phospholipiden, die eine hexagonale ($H_{II}$) Anordnung aufweisen, wenn sie unter den Testbedingungen in einem wäßrigen Medium ohne Detergens dispergiert werden;

(c) Durchführung eines lipidabhängigen diagnostischen Tests an sowohl der ersten als auch der zweiten Probe, wobei der Test ein positives Ergebnis liefert, wenn eine Lupusantikoagulanzien enthaltende Probe getestet wird; und

(d) Vergleich der Testergebnisse für die erste und die zweite' Probe, wobei das Vorliegen eines normalen Ergebnisses für die erste Probe und eines positiven Ergebnisses für die zweite Probe ein Anzeichen für die Anwesenheit von Lupusantikoagulanzien beim Patienten darstellt; wobei das Verfahren folgende schritte umfasst :

Bereitstellung der Phospholipide in Kombination mit einem für Lupustests geeigneten Detergens, die eine stabile wäßrige Suspension mit einer wäßrigen Phase, den Phospholipiden und einem Detergens bilden kann, und wobei die besagten Phospholipide in der Suspension bei einer Temperatur von 25°C mindestens eine Stunde lang suspendiert bleiben können.

9. Nachweisverfahren nach Anspruch 7 oder 8, dadurch gekennzeichnet, daß die Testprobe menschliches Plasma und das Nachweisverfahren ein Koagulationstest ist.

10. Nachweisverfahren nach Anspruch 9, dadurch gekennzeichnet, daß das Nachweisverfahren ein Verfahren zur Messung der aktivierten partiellen Thromboplastinzeit ist.

11. Nachweisverfahren nach Anspruch 7 oder 8, dadurch gekennzeichnet, daß die Phospholipide aus der Dioleoylhosphatidylethanolamin, Eier-Phosphatidylethanolamin und Rinder-Phosphatidylethanolamin umfassenden Gruppe ausgewählt werden.

12. Nachweisverfahren nach Anspruch 11, dadurch gekennzeichnet, daß das Detergens ein Salz der Desoxycholsäure enthält.

13. Nachweisverfahren nach Anspruch 12, dadurch gekennzeichnet, daß das Salz Natriumdesoxycholat ist.

14. Nachweisverfahren nach Anspruch 13, dadurch gekennzeichnet, daß das Phospholipid Dioleoylphosphatidylethanolamin ist.

**Revendications**

**Revendications pour les Etats contractants suivants : AT, BE, CH, LI, DE, DK, FR, GB, GR, IT, LU, NL, SE**

1. Une suspension aqueuse stable d'un phospholipide pour utilisation dans une méthode de dosage des anticoagulants de lupus réalisé sur un échantillon d'essai, le dosage comprenant une étape de préincubation de l'échantillon d'essai avec le phospholipide, et le phospholipide présentant une organisation hexagonale ($H_{II}$) lorsqu'il est dispersé dans un milieu aqueux sans détergent dans les conditions du dosage, la suspension comprenant :

   a) le phospholipide ;
   b) un détergent compatible avec le dosage de lupus, et
   c) une phase aqueuse,

   ledit phospholipide demeurant en suspension à une températeure de 25°C pendant au moins une heure.

2. La suspension stable de la revendication 1 où le phospholipide est sélectionné du groupe comprenant dioléoylphosphatidyléthanolamine, phosphatidyléthanolamine d'oeuf et phosphatidyléthanolamine bovine.

3. La suspension stable de la revendication 1 où ledit détergent compatible avec le dosage de lupus comprend un sel d'acide désoxycholique.

4. La suspension stable de la revendication 3, où ledit sel consiste en désoxycholate de sodium.

**5.** La suspension stable de la revendication 4 où le phospholipide consiste en dioléoylphosphatidyléthanolamine.

**6.** La suspension stable de la revendication 1, où ladite phase aqueuse consiste en une solution tampon aqueuse.

**7.** Une méthode de dosage diagnostique dépendant des lipides des anticoagulants de lupus qui est réalisée sur un échantillon d'essai, qui comprend une étape de préincubation de l'échantillon d'essai avec un ou plusieurs phospholipides qui présentent une organisation hexagonale ($H_{II}$) lorsqu'ils sont dispersés dans une phase aqueuse sans détergent dans les conditions du dosage, comprenant:

- la combinaison desdits phospholipides avec un détergent compatible avec le dosage de lupus, la combinaison du phospholipide et du détergent formant une suspension aqueuse stable lorsque mélangée avec ladite phase aqueuse dans les conditions du dosage, dans laquelle suspension lesdits phospholipides peuvent demeurer en suspension à une température de 25°C pendant au moins une heure, et
- la préincubation de l'échantillon d'essai avec ladite combinaison d'un phospholipide et d'un détergent compatible avec le dosage de lupus.

**8.** Une méthode de dosage servant à déterminer si un patient présente des anticoagulants de lupus dans lequel le dosage comprend les étapes suivantes :

a) l'obtention d'un premier et deuxième échantillons du plasma d'un patient ;
b) l'incubation du premier échantillon avec un ou plusieurs phospholipides qui présentent une organisation hexagonale ($H_{II}$) lorsqu'ils sont dispersés dans un milieu aqueux sans détergent dans les conditions du dosage ;
c) la réalisation d'un dosage diagnostique dépendant des lipides sur les premier et deuxième échantillons, le dosage produisant un relevé positif lorsqu'il est utilisé pour doser un échantillon qui contient des anticoagulants de lupus, et
d) la comparaison des résultats des dosages réalisés sur les premier et deuxième échantillons, la présence d'un résultat normal pour le premier échantillon et d'un résultat positif pour le deuxième échantillon constituant une indication que le patient présente des anticoagulants de lupus ; le dosage comprenant en outre ;

- la combinaison des phospholipides avec un détergent compatible avec le dosage de lupus qui peut former une suspension aqueuse stable comprenant une phase aqueuse, les phospholipides et un détergent, dans laquelle suspension lesdits phospholipides peuvent demeurer en suspension à une température de 25°C pendant au moins une heure.

**9.** La méthode de dosage de la revendication 7 ou 8 où l'échantillon d'essai est du plasma humain et le dosage est un test de coagulation.

**10.** La méthode de dosage de la revendication 9 où le dosage est un dosage mesurant le temps de céphaline activée.

**11.** La méthode de dosage de la revendication 7 ou 8, dans lequel les phospholipides sont choisis à même le groupe consistant en dioléoylphosphatidyléthanolamine, phosphatidyléthanolamine d'oeuf et phosphatidyléthanolamine bovine.

**12.** La méthode de dosage de la revendication 11 dans lequel le détergent comprend un sel d'acide désoxycholique.

**13.** La méthode de dosage de la revendication 12 dans lequel le sel consiste en désoxycholate de sodium.

**14.** La méthode de dosage de la revendication 13 dans lequel le phospholipide consiste en dioléoylphosphatidyléthanolamine.


**Revendications pour l'Etat contractant suivant : ES**

**1.** Méthode de dosage des anticoagulants de lupus réalisée sur un échantillon d'essai avec une suspension aqueuse stable d'un phospholipide, le dosage comprenant une étape de préincubation de l'échantillon d'essai avec le phospholipide, et le phospholipide présentant une organisation hexagonale ($H_{II}$) lorsqu'il est dispersé dans un milieu aqueux sans détergent dans les conditions du dosage, la suspension comprenant :

a) le phospholipide ;

b) un détergent compatible avec le dosage de lupus, et

c) une phase aqueuse,

ledit phospholipide demeurant en suspension à une températeure de 25°C pendant au moins une heure.

2. Méthode selon la revendication 1 où le phospholipide est sélectionné du groupe comprenant dioléoylphosphatidyléthanolamine, phosphatidyléthanolamine d'oeuf et phosphatidyléthanolamine bovine.

3. Méthode selon la revendication 1 où ledit détergent compatible avec le dosage de lupus comprend un sel d'acide désoxycholique.

4. Méthode selon la revendication 3, où ledit sel consiste en désoxycholate de sodium.

5. Méthode selon la revendication 4 où le phospholipide consiste en dioléoylphosphatidyléthanolamine.

6. Méthode selon la revendication 1, où ladite phase aqueuse consiste en une solution tampon aqueuse.

7. Une méthode de dosage diagnostique dépendant des lipides des anticoagulants de lupus qui est réalisée sur un échantillon d'essai, qui comprend une étape de préincubation de l'échantillon d'essai avec un ou plusieurs phospholipides qui présentent une organisation hexagonale ($H_{II}$) lorsqu'ils sont dispersés dans une phase aqueuse sans détergent dans les conditions du dosage, comprenant :

- la combinaison desdits phospholipides avec un détergent compatible avec le dosage de lupus, la combinaison du phospholipide et du détergent formant une suspension aqueuse stable lorsque mélangée avec ladite phase aqueuse dans les conditions du dosage, dans laquelle suspension lesdits phospholipides peuvent demeurer en suspension à une température de 25°C pendant au moins une heure, et
- la préincubation de l'échantillon d'essai avec ladite combinaison d'un phospholipide et d'un détergent compatible avec le dosage de lupus.

8. Une méthode de dosage servant à déterminer si un patient présente des anticoagulants de lupus dans lequel le dosage comprend les étapes suivantes :

a) l'obtention d'un premier et deuxième échantillons du plasma d'un patient ;

b) l'incubation du premier échantillon avec un ou plusieurs phospholipides qui présentent une organisation hexagonale ($H_{II}$) lorsqu'ils sont dispersés dans un milieu aqueux sans détergent dans les conditions du dosage ;

c) la réalisation d'un dosage diagnostique dépendant des lipides sur les premier et deuxième échantillons, le dosage produisant un relevé positif lorsqu'il est utilisé pour doser un échantillon qui contient des anticoagulants de lupus, et

d) la comparaison des résultats des dosages réalisés sur les premier et deuxième échantillons, la présence d'un résultat normal pour le premier échantillon et d'un résultat positif pour le deuxième échantillon constituant une indication que le patient présente des anticoagulants de lupus ; le dosage comprenant en outre ;

- la combinaison des phospholipides avec un détergent compatible avec le dosage de lupus qui peut former une suspension aqueuse stable comprenant une phase aqueuse, les phospholipides et un détergent, dans laquelle suspension lesdits phospholipides peuvent demeurer en suspension à une température de 25°C pendant au moins une heure.

9. La méthode de dosage de la revendication 7 ou 8 où l'échantillon d'essai est du plasma humain et le dosage est un test de coagulation.

10. La méthode de dosage de la revendication 9 où le dosage est un dosage mesurant le temps de céphaline activée.

11. La méthode de dosage de la revendication 7 ou 8, dans lequel les phospholipides sont choisis à même le groupe consistant en dioléoylphosphatidyléthanolamine, phosphatidyléthanolamine d'oeuf et phosphatidyléthanolamine bovine.

**12.** La méthode de dosage de la revendication 11 dans lequel le détergent comprend un sel d'acide désoxycholique.

**13.** La méthode de dosage de la revendication 12 dans lequel le sel consiste en désoxycholate de sodium.

**14.** La méthode de dosage de la revendication 13 dans lequel le phospholipide consiste en dioléoylphosphatidylétha-nolamine.